# EUROPÄISCHE PATENTSCHRIFT

(11) **EP 4 043 426 B1**
(45) Veröffentlichungstag und Bekanntmachung des Hinweises auf die Patenterteilung: **21.01.2026**
(21) Anmeldenummer: 21157268.0
(22) Anmeldetag: 16.02.2021
(51) Int. Cl.: C11D 17/06, C07C 41/44, C07C 43/13, C23G 5/032, C11D 7/12, C11D 17/04, C11D 7/06, C23G 5/00

(54) **VERFAHREN ZUR AUFREINIGUNG VON ALKOHOLHALTIGEN LÖSEMITTELN**
METHOD FOR PURIFYING ALCOHOL-CONTAINING SOLVENTS
PROCÉDÉ DE PURIFICATION DES SOLVANTS ALCOOLIQUES

(43) Veröffentlichungstag der Anmeldung: 17.08.2022
(73) Patentinhaber: Safechem Europe GmbH, 40474 Düsseldorf (DE)
(72) Erfinder: Rösener, Christian, 40593 Düsseldorf (DE)
(74) Vertreter: Gille Hrabal Partnerschaftsgesellschaft mbB Patentanwälte

(56) Entgegenhaltungen:
- EP-A1- 1 907 505
- EP-B1- 1 907 505
- EP-B1- 3 088 429
- WO-A1-03/062363
- WO-A1-2019/027635
- DE-A1- 2 831 210
- GB-A- 675 995

## Beschreibung

Die vorliegende Erfindung betrifft ein Verfahren zur Aufreinigung von einem alkoholhaltigen Lösemittel. Das erfindungsgemäße Verfahren findet insbesondere bei der Aufarbeitung von alkoholhaltigen Lösemitteln Anwendung, welche beispielsweise zur Reinigung, insbesondere Entfettung, von Metallteilen verwendet werden. Weiterer Gegenstand der vorliegenden Erfindung sind Zusammensetzungen, welche für den vorstehend genannten Verwendungszweck geeignet sind, sowie die Verwendung von bestimmten Zusammensetzungen zur Aufreinigung von alkoholhaltigen Lösemitteln.

GB 675 995 A offenbart, dass wässrige Lösungen von aus Olefinen hergestellten Alkoholen, die aldehydische Verunreinigungen enthalten, gereinigt werden können, indem Alkalimetallhydroxid (z.B. NaOH oder KOH) zugesetzt, mindestens eine halbe Stunde lang unter Rückfluss gehalten und anschließend der Alkohol in weitgehend aldehydfreier Form abdestilliert wird.

Bei der Entfettung von Metallteilen mittels alkoholhaltiger Lösemittel kann es zu einer Versäuerung der alkoholhaltigen Lösemittel kommen. Durch eine Versäuerung kann es im weiteren Verlauf der Verwendung zu Korrosionsschäden in der verwendeten Anlage sowie zu Beschädigungen an den zu entfettenden Metallteilen kommen.

Um diese Nachteile im Stand der Technik zu vermeiden, werden den alkoholhaltigen Lösemitteln häufig sogenannte Stabilisatoren zugesetzt, mit welchen die Säurebildung reduziert wird bzw. die gebildete Säure gebunden wird.

Aus dem Stand der Technik sind unterschiedliche Stabilisatoren bekannt, beispielsweise Zusammensetzungen auf Basis von Aminen und Kohlenwasserstoffen (vgl. EP 1 042 257 A und EP 1 907 505 A, wobei die EP 1 042 257 A die Verwendung von Aminen zur Stabilisierung nur für halogenierte Kohlenwasserstoffe, wie Perchlorethylen, und die EP 1 907 505 A Stabilisatoren für Mischungen von modifizierten Alkoholen und Kohlenwassersoffen beschreibt).

Des Weiteren offenbart EP 3 088 429 A eine Packung, enthaltend ein Reinigungsmittel verkapselt in einer wasserlöslichen Folie, wobei die Folie aus Polyvinylalkohol besteht und weitere Materialien, z.B. Polyvinylbutyral, beinhalten kann.

WO 2019/027635 A offenbart eine Reinigungsmittel-Zusammensetzung, beinhaltend Diamine (TAED) in einer Polyvinylbutyralmatrix.

WO 03/062363 A offenbart eine Kombination von Cellulasen und spezieller Cellulose in Waschmitteln.

DE 28 31 210 A offenbart ein Verfahren zur Reinigung von Ethylendiglykol durch die Verwendung von Metallhydride und Destillation.Nichtsdestotrotz ist es nach bestimmten Produktionszyklen erforderlich, das alkoholhaltige Lösemittel aufzuarbeiten oder zu verwerfen. So wird anwenderseitig zum Teil ab einer bestimmten Grenzkonzentration an Verunreinigung (Säure, Ester, etc.) das Lösemittelbad verworfen, was zu Anlagenstillstand und Kosten für Entsorgung und Neubefüllung sorgt. Für die Aufarbeitung des alkoholhaltigen Lösemittels sind neue innovative Verfahren gefragt, welche effizient sind und einfach durchgeführt werden können.

Die Aufgabe der vorliegenden Erfindung ist somit die Bereitstellung eines effizienten und einfach durchzuführenden Verfahrens zur Aufreinigung von einem alkoholhaltigen Löse- mittel, welches insbesondere bei der Entfettung von Metallteilen Anwendung findet.

Gelöst wird diese Aufgabe durch ein Verfahren zur Aufreinigung von einem alkoholhaltigen Lösemittel, welches dadurch gekennzeichnet ist, dass man in das alkoholhaltige Lösemittel ein Reinigungsmittel einträgt, welches in einer das Reinigungsmittel im Wesentlichen vollständig umgebenden Verkapselung vorliegt, wobei die Verkapselung aus einem Material besteht, das sich in dem alkoholhaltigen Lösungsmittel auflöst und es dabei nicht zu einer Bildung von störenden Verunreinigungen kommt und wobei das alkoholhaltige Lösemittel einen modifizierten Alkohol oder Mischun-gen von modifizierten Alkoholen umfasst. Das dabei verkapselte Reinigungsmittel dient zur Aufreinigung des alkoholhaltigen Lösemittels und insbesondere zur Entfernung der Versäuerung des alkoholhaltigen Lösemittels.

Im Rahmen der vorliegenden Erfindung wurde festgestellt, dass die Wirkung des Reinigungsmittels zur Aufreinigung des alkoholhaltigen Lösemittels effizient verbessert werden kann, wenn das Reinigungsmittel nicht unmittelbar in das alkoholhaltige Lösemittel eingetragen wird, sondern in einer Verkapselung vorliegt, in dieser Verkapselung in das aufzureinigende alkoholhaltige Lösemittel eingebracht wird und dort erst durch das Auflösen der Verkapselung freigesetzt wird.

Ohne an eine Theorie gebunden zu sein, wird davon ausgegangen, dass die Effizienz des erfindungsgemäß vorgesehenen Reinigungsmittels gegenüber einem unverkapselten Materials verbessert ist, da es zu einer langsamen Freisetzung des Reinigungsmittels in dem alkoholhaltigen Lösemittel kommt.

Dabei ist es im Rahmen der vorliegenden Erfindung vorgesehen, dass das Reinigungsmittel in einem Verkapselungsmaterial vorliegt, welches in dem alkoholischen Lösemittel löslich ist, so dass das im Wesentlichen verkapselte Reinigungsmittel in dem alkoholischen Lösemittel unter Auflösen der Verkapselung freigesetzt wird.

Die Geschwindigkeit der Freisetzung des Reinigungsmittels und damit die Auflösungsgeschwindigkeit der Verkapselung spielt für das erfindungsgemäße Verfahren keine wesentliche Rolle, beträgt aber unter üblichen Anwendungstemperaturen von ungefähr 100 °C 1 bis 120 Minuten, weiter bevorzugt 1 bis 60 Minuten, noch weiter bevorzugt 1 bis 30 Minuten, noch weiter bevorzugt 1 bis 10 Minuten, noch weiter bevorzugt 3 bis 5 Minuten.

Im Rahmen der vorliegenden Erfindung kommt es vorzugsweise auf die richtige Auswahl eines geeigneten Verkapselungsmaterials an. Das erfindungsgemäß vorgesehene Verkapselungsmaterial muss sich zunächst einmal überhaupt in dem alkoholhaltigen Lösemittel, gegebenenfalls bei erhöhter Temperatur, auflösen. Eine weitere Anforderung, die im Rahmen der vorliegenden Erfindung an das Verkapselungsmaterial gestellt wird, ist, dass das Verkapselungsmaterial sich in dem alkoholhaltigen Lösemittel auflöst und es dabei nicht zu einer Bildung von störenden Verunreinigungen kommt. Darüber hinaus ist es für die vorliegende Erfindung von Vorteil, wenn das Verkapselungsmaterial einfach hergestellt werden kann und eine einfache und effiziente, möglichst vollständige Umhüllung des erfindungsgemäßen Reinigungsmittels ermöglicht.

Stabilisatoren, wie die aus dem Stand der Technik bekannten, liegen in flüssiger Form vor und bestehen aus Mischungen von Flüssigkeiten bzw. aus in Flüssigkeiten gelösten Feststoffen.

Pulverförmige Substanzen werden dagegen bisher nicht großflächig eingesetzt. Diese können eine Staubexplosion hervorrufen, wenn der Stoff in der Luft verwirbelt wird und Zündquellen vorhanden sind. Bei Stäuben ist die Vermeidung explosionsfähiger Gemische durch Begrenzung der Staubkonzentration dabei schwer zu erreichen.

Durch die Verkapselung des pulverförmigen Reinigungsmittels wird daher eine neuartige Möglichkeit der Aufreinigung alkoholhaltiger Lösemittel geschaffen, da eine gefährliche Verwirbelung der Substanz in der Luft durch die Verkapselung verhindert wird.

Dabei sollte das Verkapselungsmaterial möglichst nicht toxisch sein, so dass die Anwender des erfindungsgemäßen Verfahrens das Verkapselungsmaterial ohne Explosionsschutzmaßnahmen und geringen Sicherheitsmaßnahmen zum Lösemittel zugeben können - d.h. ohne Tragen von persönlicher Schutzausrüstung zur Vermeidung der dermalen und inhalativen Exposition. Des Weiteren ist es für die Auswahl eines geeigneten Verkapselungsmaterial von Vorteil, wenn das Material transparent ausgebildet ist, so- dass ein in Augenschein nehmen des Reinigungsmittels vor dessen Anwendung in dem alkoholhaltigen Lösemittel auf einfache Weise möglich ist.

Schließlich sollte das Verkapselungsmaterial unterschiedliche Mengen an Reinigungsmittel enthalten können, sodass eine entsprechende Dosierung durch den Anwender des erfindungsgemäßen Verfahrens auf einfache Weise ausgeführt werden kann.

Im Rahmen der vorliegenden Erfindung wurden zahlreiche unterschiedliche Verkapselungsmaterialien für diesen Verwendungszweck untersucht und geeignete Materialien für die Ausbildung der Verkapselung sind beispielsweise Cellulosederivate, insbesondere Celluloseester, wie Cellulosenitrat, Celluloseacetat oder Celluloseacetatbutyrat, sowie Polyvinylbutyral.

Bezüglich den Cellulosederivaten kann der Fachmann auf Basis des Löslichkeitsverhaltens, der Art der Substituenten, des durchschnittlichen Substitutionsgrads, der Substitutionsverteilung in der Kette und deren Verteilung auf die primären und sekundären Alkoholgruppen für unterschiedliche alkoholhaltige Lösemittel geeignete Cellulosederivate auffinden, die in alkoholhaltigen Lösemitteln unter den erfindungsgemäß anzuwendenden Bedingungen löslich sind.

Ein weiteres geeignetes Material für die Ausbildung der Verkapselung ist Polyvinylbutyral (PVB). Polyvinylbutyrale mit hoch acetalisierten Einstellungen (hoher Acetalisierungsgrad) sind insbesondere alkohollöslich. Für die Ausbildung des Verpackungsmaterials ist es dabei insbesondere bevorzugt, wenn das Polyvinylbutyral zu Folien mit einer geringen dynamischen Viskosität verarbeitet werden kann. Entsprechende handelsübliche Ausgangsmaterialien für die Herstellung der Verkapselung werden beispielsweise von der Firma Kuraray Europe GmbH kommerziell vertrieben. Geeignete Handelsprodukte sind beispielsweise Mowital^{®} B30H oder Mowital^{®} B20H.

Im Rahmen der vorliegenden Erfindung ist als Verkapselungsmaterial Polyvinylbutyral insbesondere bevorzugt. Die Gründe hierfür werden nachfolgend erläutert:
Zunächst einmal ist Polyvinylbutyral thermoplastisch verarbeitbar, lichtstabil und bei Temperaturen oberhalb von 120 °C heißsiegelfähig. Daher ist es im Rahmen der vorliegenden Erfindung einfach, Folienmaterialien zur Herstellung von verkapselten Reinigungsmitteln aus diesen Verkapselungsmaterialien, beispielsweise in der Form von Beuteln, herzustellen.

Die gewünschte Verkapselung auf Basis von Polyvinylbutyral weist darüber hinaus den Vorteil auf, dass die resultierende Verkapselung klar und transparent ist, sodass ein in Augenschein nehmen des verkapselten Reinigungsmittels vor dessen Anwendung zur Behandlung des alkoholhaltigen Lösemittels möglich ist.

Ein weiterer wesentlicher Vorteil bei der Verwendung von Polyvinylbutyral als Verkapselungsmaterial ergibt sich dadurch, dass sich das Polyvinylbutyral einfach unter den angewendeten Verfahrensbedingungen auflöst und dabei keine störenden Nebenprodukte in das aufzugreinigende alkoholhaltige Lösemittel eingetragen werden.

In einer bevorzugten Ausführungsform der vorliegenden Erfindung umfasst das Verkapselungsmaterial nicht nur Polyvinylbutyral und andere Polymere, sondern besteht im Wesentlichen, insbesondere ausschließlich, aus Polyvinylbutyral. Im Rahmen der vorliegenden Erfindung ist in einer bevorzugten Ausführungsform daher ein Verkapselungsmaterial bestehend aus Polyvinylbutyral vorgesehen.

Im Nachfolgenden werden besonders bevorzugte Ausgestaltungen des erfindungsgemäß bevorzugten Polyvinylbutyrals als Verkapselungsmaterial beschrieben.

Das im Rahmen der vorliegenden Erfindung vorzugsweise verwendete Polyvinylbutyral weist im Allgemeinen einen Gehalt an Polyvinylalkohol von 11 bis 27 Gew.-%, bezogen auf die Gesamtmasse an Polyvinylbutyral, auf.

Dabei ist es weiter und unabhängig davon bevorzugt, wenn das Polyvinylbutyral einen Gehalt an Polyvinylacetat aufweist. Weiter bevorzugt ist es, wenn der Gehalt an Polyvinylacetat in dem Polyvinylbutyral bis 8 Gew.-%, bezogen auf die Gesamtmasse an Polyvinylbutyral, beträgt.

Unabhängig von den vorstehenden Eigenschaften des bevorzugt zu verwendenden Polyvinylbutyrals weist dieses Verkapselungsmaterial vorzugsweise eine dynamische Viskosität von 9 bis 330 mPa • s in einer 10%igen Lösung in Ethanol auf.

Unabhängig von den vorstehenden Eigenschaften des bevorzugt zu verwendenden Polyvinylbutyrals weist dieses Verkapselungsmaterial vorzugsweise eine Wasseraufnahme-Kapazität des Polyvinylbutyrals nach 24 Stunden bei 20 °C von 200 bis 340 g/l auf.

Unabhängig von den vorstehenden Eigenschaften des bevorzugt zu verwendenden Polyvinylbutyrals weist dieses Verkapselungsmaterial vorzugsweise eine Glasübergangstemperatur, bestimmt nach DSC, ISO11357-1, von 60 bis 92 °C auf.

Unabhängig von den vorstehenden Eigenschaften des bevorzugt zu verwendenden Polyvinylbutyrals weist dieses Verkapselungsmaterial vorzugsweise einen Feststoffgehalt von mehr als 97,5 Gew.-% auf.

Die vorstehend beschriebenen Eigenschaften des Polyvinylbutyrals machen das Polyvi- nylbtyral besonders geeignet, im erfindungsgemäßen Verfahren als Kapselmaterial verwendet zu werden.

Erfindungsgemäß besonders bevorzugte Ausführungsformen der vorliegenden Erfindung verwenden Polyvinylbutyral, welches mehr als eine der vorstehend beschriebenen Eigenschaften aufweist. Insbesondere weist das erfindungsgemäß zu verwendende Polyvinylbutyral mindestens 2, weiter bevorzugt mindestens 3, weiter bevorzugt mindestens **4,** weiter bevorzugt mindestens 5, der zuvor beschriebenen Eigenschaften auf.

Das im Rahmen der vorliegenden Erfindung in der Verkapselung vorliegende Reinigungsmittel umfasst vorzugsweise eine Base. Mit Hilfe der Base als Reinigungsmittel werden saure Verunreinigungen, die sich während der Verwendung des alkoholhaltigen Lösemittels ergeben, neutralisiert und somit für die weitere Verwendung des alkoholhaltigen Lösemittels unschädlich gemacht.

In einer bevorzugten Ausführungsform besteht das Reinigungsmittel im Wesentlichen, insbesondere ausschließlich, aus einer Base.

Auch die Verwendung von mehreren Basen als Reinigungsmittel ist von der vorliegenden Erfindung umfasst.

Darüber hinaus kann das Reinigungsmittel weitere Bestandteile aufweisen, die ebenfalls zur Reinigung des alkoholhaltigen Lösemittels verwendet werden.

Sollte im Rahmen der vorliegenden Erfindung eine Base als Reinigungsmittel verwendet werden, so weist das alkoholhaltige Lösemittel als Verunreinigung vorzugsweise eine Säure auf. Darüber hinaus können sich aus der als Verunreinigung vorliegenden Säure auch unspezifische Veresterungsprodukte bilden, welche durch das plastische Reinigungsmittel aus dem alkoholhaltigen Lösemittel entfernt werden.

In einer weiteren Ausführungsform der vorliegenden Erfindung ist das Reinigungsmittel, welches in der Verkapselung verwendet wird, hygroskopisch. Durch die Verwendung eines hygroskopischen Reinigungsmittels lassen sich neben sauren Verunreinigungen auch wässrige Verunreinigungen durch das erfindungsgemäße Verfahren effizient abtrennen. Wässrige Verunreinigungen können sich beispielsweise durch unspezifische Esterbildungen von der Säureverunreinigungen mit dem Alkohol in dem alkoholhaltigen Lösemittel bilden.

Die Verwendung von einem hygroskopischen Reinigungsmittel sowie das Vorliegen von Wasser als Verunreinigung in dem alkoholischen Lösemittel ist für die vorliegende Erfindung jedoch nicht zwingend erforderlich.

In einer bevorzugten Ausführungsform der vorliegenden Erfindung ist das Reinigungsmittel vorzugsweise eine Base aus der Gruppe der Alkali- oder Erdalkalihydroxide sowie Mischungen davon. In einer bevorzugten Ausführungsform der vorliegenden Erfindung wird das Reinigungsmittel ausgewählt aus der Gruppe, bestehend aus Natriumcarbonat, Kaliumcarbonat, Natriumhydroxid, Kaliumhydroxid, Natriumhydrogencarbonat, Calciumhydroxid, Magnesiumcarbonat und Mischungen der vorstehenden Reinigungsmittel.

In einer weiteren bevorzugten Ausführungsform der vorliegenden Erfindung wird das Reinigungsmittel ausgewählt aus der Gruppe, bestehend aus bestehend aus Natriumcarbonat, Kaliumcarbonat, Natriumhydroxid, Kaliumhydroxid und Mischungen der vorstehenden Reinigungsmittel.

Nach dem in Kontakt bringen des aufzureinigenden alkoholhaltigen Lösemittels mit dem verkapselten Reinigungsmittel, wird im Rahmen der vorliegenden Erfindung das aufzureinigende alkoholhaltigen Lösemittel destilliert (refluxiert).

Diese Destillation bzw. diese Refluxion des alkoholhaltigen Lösemittels wird vorzugsweise für einen Zeitraum von mindestens 1 Stunde, vorzugsweise mindestens 2 Stunden, weiter bevorzugt mindestens 3 Stunden, weiter bevorzugt mindestens 6 Stunden, weiter bevorzugt mindestens 12 Stunden, weiter bevorzugt mindestens 24 Stunden, durchgeführt. Aufgrund der verwendeten Temperaturen in Kombination mit dem verwendeten Reinigungsmittel kommt es zu einer effizienten Entfernung der Verunreinigungen aus dem alkoholhaltigen Lösemittel.

In der Praxis kann das erfindungsgemäße Verfahren dadurch durchgeführt werden, dass das verkapselte Reinigungsmittel dem Sumpf einer mit dem aufzureinigenden Lösemittel versehenen Destillationseinheit (Refluxiervorrichtung) zugegeben wird und dann bei den erfindungsgemäß vorgesehenen Temperaturen verwendet wird.

Alternativ ist es auch möglich, dass verkapselte Reinigungsmittel an einer beliebigen anderen Stelle der verwendeten Einheit bzw. Vorrichtung zuzuführen. Beliebige andere Stellen sind beispielsweise ein verwendeter Wasserabscheider.

Im Rahmen der vorliegenden Erfindung hat es sich jedoch technisch am sinnvollsten erwiesen, dass das verkapselte Reinigungsmittel zum Sumpf zugegeben wird, da ein Feststoff abgetrennt werden muss.

Im Rahmen der vorliegenden Erfindung kann neben dem verkapselten Reinigungsmittel mindestens ein oder mehrere Antioxidantien verwendet werden. Dieses Antioxidant wird dem alkoholhaltigen Lösemittel vor oder während dem Inkontaktbringen mit dem verkapselten Reinigungsmittel zugesetzt.

Geeignete Antioxidantien, die während dem erfindungsgemäßen Verfahren zur Aufreinigung des alkoholhaltigen Lösemittels zusätzlich verwendet werden können, sind beispielsweise phenolische Antioxidantien, aminhaltige Antioxidantien, Antioxidantien auf Basis organischer Schwefelverbindungen, Antioxidantien auf Basis von organischen Phosphorverbindungen und Mischungen der zuvor genannten Antioxidantien.

Entsprechende Antioxidantien sind dem Fachmann aus Ullmann's Encyclopedia of industrial chemistry, Kapitel: Antioxidants (2015, Wiley-VCH Verlag GmbH & Co. KG, Weinheim) bekannt. Insbesondere geeignete Antioxidantien lassen sich den Kapiteln 4.2, 4.3, 4.4 und 4.5 entnehmen.

In einer ersten Ausführungsform ist das Antioxidant ein phenolisches Antioxidant. In einer zweiten Ausführungsform ist das Antioxidant ein aminhaltiges Antioxidant.

In einer dritten Ausführungsform ist das Antioxidant ein Antioxidant auf Basis organischer Schwefelverbindungen.

In einer vierten Ausführungsform ist das Antioxidant ein Antioxidant auf Basis von organischen Phosphorverbindungen.

Besonders geeignete Antioxidantien werden ausgewählt aus der Gruppe, bestehend aus 2,6-Di-tert.-butyl-4-methylphenol, 2,6-Di-tert.-butylphenol und Mischungen davon.

Weitere geeignete Antioxidantien werden ausgewählt aus der Gruppe, bestehend aus p,p'-Dioctyl-diphenylamin, oligomerischem 1,2-Dihydro-2,2,4-trimethylchinolin, 1-Naphthylphenylamin, Derivaten des Diphenylamins und Mischungen davon.

Diese Antioxidantien werden vorzugsweise zusammen mit dem Reinigungsmittel in der das Reinigungsmittel im Wesentlichen vollständig umgebenden Verkapselung verwendet oder dem alkoholhaltigen Lösemittel separat zugegeben.

In einerweiteren Ausgestaltung der vorliegenden Erfindung wird das Reinigungsmittel verkapselt gegebenenfalls in Kombination mit mindestens einem Antioxidant sowie zusätzlich mit Kieselgel als oberflächenaktive Substanz verwendet.

Das erfindungsgemäße Verfahren findet insbesondere bei alkoholhaltigen Lösemitteln Anwendung, die aus (Metal-)Entfettungsanlagen resultieren. Bei (Metall-)Entfettungsanlagen handelt es sich um geschlossene Systeme, welche eine Reinigungskammer, eine unterschiedliche Anzahl an Lösemitteltanks sowie eine Destillationseinrichtung umfassen. Mittels eines Lösemittels, bevorzugt mit weiter untenstehend noch definierten modifizierten Alkoholen, werden verunreinigte Teile in der Reinigung gereinigt.

Das verunreinigte, also mit den abgelösten Rückständen der gereinigten und entfetteten Bauteile angereicherte, alkoholhaltigen Lösemittel fließt anschließend wieder zurück in die Lösemitteltanks. Diese Tanks speisen die in der Entfettungsanlage integrierte Destillationseinrichtung permanent mit dem verunreinigten Lösemittel. Während des Destillationsprozesses wird das früher beziehungsweise bei geringerer Temperatur siedende Lösemittel von den zuvor abgereinigten Rückständen getrennt. Bei diesem Destillationsprozess kann es zu einer Versäuerung durch Selbstzersetzung des Lösemittels kommen. Beispielsweise können beim Einsatz von modifiziertem Alkohol als Lösemittel leichtsiedende Bestandteile und vor allem Säuren entstehen. Die Versäuerung kann wiederum zu Korrosionsschäden in der Reinigungsanlage und zur Oberflächenkorrosion auf den gereinigten Teilen führen.

Um der Säurebildung in der Destillationseinrichtung entgegenzuwirken, wird dem Destillationsprozess das erfindungsgemäß vorgesehene verkapselte Reinigungsmittel während der Aufreinigung zugegeben. Das Reinigungsmittel bindet im Destillationssumpf die entstandenen Säuren und verbleibt im Destillationssumpf. Das Reinigungsmittel verbleibt also während des gesamten Reinigungs- und Aufbereitungsprozesses des Löse- mittels in der Destillationseinrichtung.

Das Destillat, also das destillierte und gereinigte alkoholhaltigen Lösemittel, wird unter anderem anschließend über einen Wasserabscheider zurück in die Lösemitteltanks geführt. Der Destillationssumpf wird in regelmäßigen Abständen geleert und entsorgt. Das somit verbrauchte Reinigungsmittel zusammen mit möglichen nicht störenden Rückständen aus dem Material der Verkapselung, wird zusammen mit den abgereinigten Rückständen der Bauteile ausgetragen.

Mittels eines Schnelltestverfahrens zur Lösemittelüberprüfung kann der Anwender die Qualität, also den Säuregehalt, des eingesetzten Lösemittels direkt selbst an der Anlage bestimmen.

Unter Kenntnis der ermittelten Acidität des alkoholhaltigen Lösemittels, also des Anteils an freie Säuren im alkoholhaltigen Lösemittel, und des Anlagenvolumens der Entfettungsanlage kann mittels stöchiometrischer Kalkulation die benötigte Zugabemenge des erfindungsgemäßen Reinigungsmittels ermittelt werden, um den Säuregehalt zu neutralisieren.

Im Rahmen der vorliegenden Erfindung handelt es sich bei dem alkoholhaltigen Lösemittel im Allgemeinen um einzelne modifizierte Alkohole oder Mischungen von modifizierten Alkoholen.

Modifizierte Alkohole sind beispielsweise Propanole, denen eine Ethergruppe zugefügt wurde (Alkoxy-Propanole). Durch die Variation der Ethergruppe kann das Lösevermögen der modifizierten Alkohole der Art der Verunreinigung (Entfettung) angepasst werden.

Bei den modifizierten Alkoholen handelt es sich beispielsweise um Glykolether-Mischun-gen und/oder Glykolether-Kohlenwasserstoff-Mischungen.

Besonders bevorzugt sind im Rahmen der vorliegenden Erfindung 1-Butoxy-2-propanol und Butoxy-1-propanol. Auch Mischungen dieser beiden zuvor genannten modifizierten Alkohole sind im Rahmen der vorliegenden Erfindung besonders bevorzugt.

In einem weiteren Aspekt betrifft die vorliegende Erfindung eine Zusammensetzung aus einem Reinigungsmittel und einer Verkapselung, die vorzugsweise in dem vorstehend beschriebenen erfindungsgemäßen Verfahren Anwendung findet.

Daher betrifft die vorliegende Erfindung auch eine Zusammensetzung, enthaltend ein Reinigungsmittel in einer Verkapselung aus einem Polymermaterial, enthaltend Polyvinylbutyral, wobei das Reinigungsmittel in der Verkapselung Natriumcarbonat, Kaliumcarbonat, Natriumhydroxid, Kaliumhydroxid und Mischungen der vorstehenden Reinigungsmittel umfasst oder daraus besteht.

Gemäß den vorstehenden Erläuterungen zu dem erfindungsgemäßen Verfahren umfasst das Reinigungsmittel in der Verkapselung vorzugsweise eine Base oder besteht im Wesentlichen, insbesondere ausschließlich, aus einer Base.

Die Verkapselung umgibt das Reinigungsmittel im Wesentlichen vollständig.

Wie bereits vorstehend ausgeführt ist es bevorzugt, wenn das Reinigungsmittel in der Verkapselung hygroskopisch ist.

In einer bevorzugten Ausführungsform der vorliegenden Erfindung ist die Zusammensetzung dadurch gekennzeichnet, dass das Reinigungsmittel ausgewählt wird aus der Gruppe, bestehend aus Natriumcarbonat, Kaliumcarbonat, Natriumhydroxid, Kaliumhydroxid, Natriumhydrogencarbonat, Calciumhydroxid, Magnesiumcarbonat und Mischungen der vorstehenden Reinigungsmittel.

In einer weiteren bevorzugten Ausführungsform der vorliegenden Erfindung wird das Reinigungsmittel ausgewählt aus der Gruppe, bestehend aus bestehend aus Natriumcarbonat, Kaliumcarbonat, Natriumhydroxid, Kaliumhydroxid und Mischungen der vorstehenden Reinigungsmittel.

Wie bereits vorstehend ausgeführt umfasst das Kapselmaterial vorzugsweise Polyvinylbutyral.

In der weiteren bevorzugten Ausführungsform der vorliegenden Erfindung besteht das Kapselmaterial im Wesentlichen, insbesondere ausschließlich, aus Polyvinylbutyral.

Für den angegebenen Verwendungszweck zur Entfettung von Metallteilen gemäß dem obenstehenden Verfahren ist es des Weiteren bevorzugt, wenn das Polyvinylbutyral einen Gehalt an Polyvinylalkohol von 11 bis 27 Gew.-%, bezogen auf die Gesamtmasse an Polyvinylbutyral aufweist.

In einer weiteren Ausgestaltung ist die erfindungsgemäße Zusammensetzung dadurch gekennzeichnet, dass das Polyvinylbutyral einen Gehalt an Polyvinylacetat aufweist.

Wenn das Polyvinylbutyral in der erfindungsgemäßen Zusammensetzung einen Gehalt an Polyvinylacetat aufweist, so beträgt der Gehalt an Polyvinylacetat in dem Polyvinylbutyral vorzugsweise bis 8 Gew.-%, bezogen auf die Gesamtmasse an Polyvinylbutyral.

Die dynamische Viskosität des erfindungsgemäß zu verwendenden Polyvinylbutyrals beträgt vorzugsweise 9 bis 330 mPa • s in einer 10%igen Lösung in Ethanol.

Die Wasseraufnahme-Kapazität des erfindungsgemäß zu verwendenden Polyvinylbutyrals beträgt nach 24 Stunden bei 20 °C vorzugsweise 200 bis 340 g/l.

Die Glasübergangstemperatur des erfindungsgemäß zu verwendenden Polyvinylbutyrals, bestimmt nach DSC, ISO11357-1, beträgt vorzugsweise 60 bis 92 °C beträgt.

Der Feststoffgehalt des erfindungsgemäß zu verwendenden Polyvinylbutyrals beträgt vorzugsweise mehr als 97,5 Gew.-%.

In der erfindungsgemäßen Zusammensetzung kann neben dem eigentlichen Reinigungsmittel, die vorstehend näher beschriebene Base, noch mindestens ein oder mehrere Antioxidantien verwendet werden.

Entsprechende Antioxidantien sind dem Fachmann aus Ullmann's Encyclopedia of industrial chemistry, Kapitel: Antioxidants (2015, Wiley-VCH Verlag GmbH & Co. KG, Weinheim) bekannt. Insbesondere geeignete Antioxidantien lassen sich den Kapiteln 4.2, 4.3, **4.4** und 4.5 entnehmen.

In einer ersten Ausführungsform ist das Antioxidant ein phenolisches Antioxidant.

In einer zweiten Ausführungsform ist das Antioxidant ein aminhaltiges Antioxidant.

In einer dritten Ausführungsform ist das Antioxidant ein Antioxidant auf Basis organischer Schwefelverbindungen.

In einer vierten Ausführungsform ist das Antioxidant ein Antioxidant auf Basis von organischen Phosphorverbindungen.

Besonders geeignete Antioxidantien werden ausgewählt aus der Gruppe, bestehend aus 2,6-Di-tert.-butyl-4-methylphenol, 2,6-Di-tert.-butylphenol und Mischungen davon.

Weitere geeignete Antioxidantien werden ausgewählt aus der Gruppe, bestehend aus p,p'-Dio-ctyldiphenylamin, oligomerischem 1,2-Dihydro-2,2,4-trimethylchinolin, 1-Naphthylphenylamin, Derivaten des Diphenylamins und Mischungen davon.

Des Weiteren betrifft die vorliegende Erfindung die Verwendung der oben beschriebenen Zusammensetzung zur Aufreinigung von einem Lösemittel, dass insbesondere zur Entfettung von Metallteilen verwendet wird. Es wird auf obige Ausführungen verwiesen.

Des Weiteren betrifft die vorliegende Erfindung ein Verfahren zur Herstellung einer erfindungsgemäßen Zusammensetzung, die in ihren Einzelheiten oben beschrieben wurde. Das erfindungsgemäße Verfahren ist durch die nachfolgenden Verfahrensschritte:
i. Bereitstellung einer Folie, umfassend Polyvinylbutyral;
ii. Bereitstellung eines Reinigungsmittels;
iii. Einschweißen des Stoffs in die Folie, umfassend Polyvinylbutyral, so dass der Stoff in einer im Wesentlichen vollständig umgebenden Verkapselung vorliegt.

In diesem Verfahren wird die Verkapselung durch eine Verschweißung der Folie bei einer Temperatur von mindestens 120 °C durchgeführt.

Für weitere Einzelheiten wird auf obige Ausführungen verweisen.

Die nachfolgenden Beispiele verdeutlichen die vorliegende Erfindung im Detail:

### Ausführungsbeispiele

### Versuch 1: Einfacher Mischversuch und Abdampfverhalten

Eine Folie Mowital Thin Film 100 wird mit Lösemittel (DOWCLENE^{®} 1601) bei Raumtemperatur versetzt; anschließend wird das Lösemittel verdampft.

Analytische Ergebnisse:
1. Löslichkeit: Ohne Agitation wurde nach ca. 30 Minuten eine vollständige Auflösung des Kunststoffs beobachtet.
2. Aussehen nach Abdampfen: Das Lösemittel wurde über Nacht bei 80 °C verdampft. Im Behältnis bildete sich ein einheitlicher, farbloser Mowital-Film.

Bewertung:
Die Löslichkeit der Folie ist gut und die Rückgewinnung zeigt, dass der Kunststoff durch die Kombination aus Temperatur und Lösemittel keine offensichtlichen Abbaureaktionen zeigt.

### Versuch 2: Dauerversuch in Labor-Destillationsapparatur mit frischem DOWCLENE^{®} 1601

Mowital Thin Film 100 in Lösemittel (DOWCLENE^{®} 1601) werden im Destillationssumpf der Laborapparatur vermischt. In den Dampfraum der Apparatur werden Metallstreifen aus Aluminium, Stahl, Kupfer und Messing eingehängt. Das Lösemittel wird im Unter- druck (100 mbar) für 6 Tage durchgängig refluxiert.

Analytische Ergebnisse:
1. GC/FID-Analyse des Destillats: Es wurde Lösemittel in unveränderter Reinheit zurückgewonnen. Flüchtige organische Abbauprodukte aus dem Kunststoff wurden nicht identifiziert.
2. Säuregehalt des Destillats (Titrationsmethode): Das Lösemittel zeigte sich unverändert und im Rahmen der Spezifikation.
3. Optische Prüfung der Metallstreifen: Die Metallstreifen zeigten keine Beschichtung, oder ähnliche kunststoffartigen Niederschläge.
4. Beurteilung des Sumpfs: Der Sumpf zeigte sich viskos, was durch die hohe Konzentration an PVB ausgelöst wurde. Eine Verklumpung oder Anbackung des Kunststoffs wurde nicht beobachtet. Die Farbe zeigte sich unverändert.
5. FT-IR Analyse des Sumpfs: Nach dem Versuch wurde das Lösemittel im Destillationsrückstand verdampft. Ein FT-IR Spektrum des im Sumpf verbleibenden Kunststoffs wurde aufgenommen und mit einer unbenutzten Kunststofffolie verglichen. Hier zeigte sich keine Änderung zwischen dem aufgelösten, erhitzen Kunststoff nach dem Versuch und der neuwertigen Folie.

### Bewertung:

Es zeigen sich selbst bei dauerhaft hoher Temperatur im siedenden Lösemittel keine potentiell störenden Abbauprodukte. Der Kunststoff wurde vom Lösemittel gelöst, zeigte sich aber chemisch unverändert. Das aus dem Sumpf destillierte Lösemittel entspricht der eingesetzten Frischware und ist uneingeschränkt zur Reinigung verwendbar.

### Versuch 3: Dauerversuch in Labor-Destillationsapparatur mit saurem DOWCLENE^{®} 1601

Der oben genannte Versuch 2 wurde mit Mowital-Granulat B30H und mit saurem Lösemittel (Säurezahl ca. 6000 ppm) wiederholt.

### Analytische Ergebnisse:

1. GC/FID-Analyse des Destillats: Es wurde ausschließlich Lösemittel mit unveränderter Qualität zurückgewonnen. Flüchtige organische Abbauprodukte aus dem Kunststoff wurden nicht identifiziert.
2. Säuregehalt des Destillats (Titrationsmethode): Die Säurezahl blieb konstant.
3. Optische Prüfung der Metallstreifen: Die Metallstreifen zeigten keine Beschichtung, oder ähnliche kunststoffartigen Niederschläge.
4. Beurteilung des Sumpfs: Der Sumpf zeigte eine Verfärbung. Dies ist bei Destillation von säurehaltigen Proben häufig zu beobachten. Eine Verklumpung oder Anbackung des Kunststoffs wurde nicht beobachtet.

### Bewertung:

Die für frisches Lösemittel erarbeitete Erkenntnis zeigt sich auch bei säurehaltigem Lösemittel: Es wurden keine Abbauprodukte des Kunststoffs beobachtet; das Lösemitteldestillat entspricht in der Qualität dem eingesetzten Lösemittel und ist zur Reinigung einsetzbar.

### Versuch 4: Säurereduktion mithilfe verkapseltem Reinigungsmittel - Probe 1

Säurehaltiges DOWCLENE 1601 (aus Kunden-Reinigungsanlage entnommenes Lösemittel als möglichst realistisches Beispiel) werden über Nacht bei 100 mbar refluxiert. In den Dampfraum der Apparatur werden Metallstreifen aus Aluminium, Stahl, Kupfer und Messing eingehängt. Am nächsten Tag wird an der Probenahmstelle im Destillat eine Probe zur Nullwertbestimmung genommen.

Das verkapselte Reninigungsmittel wird durch das Einschweißen von wasserfreiem Natriumcarbonat und Beimengen eines Antioxidants in Mowital Thin Film 100-Folie hergestellt.

Zur Reduktion der Säure wird das verkapselte Reinigungsmittel zum Sumpf hinzugefügt. Die Folie löst sich in heißem Lösemittel innerhalb von 3 bis 5 Minuten auf und gibt den Feststoff frei, der sich feindispers verteilt.

Das Lösemittel wird für weitere 6 Stunden refluxiert, wobei das Reinigungsmittel die Säure im Sumpf komplexiert. Zur Erfolgsbeurteilung werden nach 2, 4 und 6 Stunden weitere Proben genommen. Die Destillationsleistung der Apparatur beträgt ca. 1/3 des Gesamt-Lösemittelvolumens pro Stunde.

### Analytische Ergebnisse:

1. Säuregehalt des Destillats (Titrationsmethode):
   - Nullwert: 9853 ppm als Essigsäure
   - Nach 2 Std (2/3fache Umwälzung des Volumens): 2761 ppm
   - Nach 4 Std (4/3fache Umwälzung des Volumens): 1510 ppm
   - Nach 6 Std (2fache Umwälzung des Volumens): 578 ppm
2. GC/FID-Analyse: Das Destillat zeigte sich an allen Probenahmezeitpunkten in gleichbleibender Qualität, mit der Ausnahme, dass das Essigsäure-Signal nach 6 Stunden einen mit dem Titrationsergebnis einhergehenden Abfall in der Intensität aufwies.
3. lonenchromatographische Analyse des Destillats nach 2 Stunden: Eine zur Säurekonzentration (2761 ppm) passende Konzentration an Acetat und Formiat wurde gemessen (2190 mg/L Acetat, 440 mg/L Formiat). Weitere Anionen wurden nicht identifiziert.
4. Optische Prüfung der Metallstreifen: Die Metallstreifen zeigten keine Beschichtung oder ähnliche kunststoffartigen Niederschläge.
5. Beurteilung des Sumpfs: Der Sumpf zeigte feindispers verteilten, weißen Feststoff. Eine Verklumpung oder Anbackung des Feststoffs oder Kunststoffs wurde nicht beobachtet. Der Feststoff ist wasserlöslich.

### Bewertung:

Das verkapselte Reinigungsmittel zeigte eine deutliche Reduktion der Säurezahl nach bereits kurzer Versuchsdauer. Der Feststoff war nicht verklumpt und eine Entsorgung des Feststoffs war durch die Wasserlöslichkeit einfach möglich.

Das Lösemitteldestillat zeigt sich unverändert zum Ausgangsmaterial, mit der Ausnahme, dass die Säure entfernt wurde. Das Lösemittel kann somit weiter zur Reinigung eingesetzt werden und gewinnt durch die Säureentfernung deutlich an Wertigkeit, da korrosive Bestandteile entfernt werden.

### Versuch 5: Säurereduktion mithilfe verkapseltem Reinigungsmittel - Probe 2

Säurehaltiges DOWCLENE^{®} 1601 (aus Kunden-Reinigungsanlage entnommenes Lösemittel als möglichst realistisches Beispiel) werden für 2 Stunden bei 100 mbar refluxiert. In den Dampfraum der Apparatur werden Metallstreifen aus Aluminium, Stahl, Kupfer und Messing eingehängt. Nach 2 Stunden wird an der Probenahmstelle im Destillat eine Probe zur Nullwertbestimmung genommen.

Das verkapselte Reinigungsmittel wird durch das Einschweißen von wasserfreiem Natriumcarbonat und Beimengen eines Antioxidants in Mowital Thin Film 100-Folie hergestellt.

Zur Reduktion der Säure wird das verkapselte Reinigungsmittel zum Sumpf hinzugefügt. Die Folie löst sich in heißem Lösemittel innerhalb von 3 bis 5 Minuten auf und gibt den Feststoff frei, der sich feindispers verteilt.

Das Lösemittel wird für weitere 24 Stunden refluxiert, wobei das Reinigungsmittel die Säure im Sumpf komplexiert. Zur Erfolgsbeurteilung werden nach 2 und 24 Stunden weitere Proben genommen. Die Destillationsleistung der Apparatur beträgt ca. 1/3 des Gesamt-Lösemittelvolumens pro Stunde.

### Analytische Ergebnisse

1. Säuregehalt des Destillats (Titrationsmethode):
   - Nullwert: 6303 ppm als Essigsäure
   - Nach 2 Std (2/3fache Umwälzung des Volumens): 3009 ppm
   - Nach 24 Std (8fache Umwälzung des Volumens): <10 ppm
2. GC/FID-Analyse: Das Destillat zeigte sich an allen Probenahmezeitpunkten in gleichbleibender Qualität, mit der Ausnahme, dass das Essigsäure-Signal nach 24 Stunden einen mit dem Titrationsergebnis einhergehenden Abfall in der Intensität aufwies.
3. lonenchromatographische Analyse des Destillats nach 2 Stunden: Eine zur Säurekonzentration (3009 ppm) ungefähr passende Menge an Acetat und Formiat wurde identifiziert (1550 mg/L Acetat, 940 mg/L Formiat). Weitere Anionen wurden nicht identifiziert.
4. Optische Prüfung der Metallstreifen: Die Metallstreifen zeigten keine Beschichtung oder ähnliche kunststoffartigen Niederschläge.
5. Beurteilung des Sumpfs: Der Sumpf zeigte feindispers verteilten, weißen Feststoff. Eine Verklumpung oder Anbackung des Feststoffs oder Kunststoffs wurde nicht beobachtet. Der Feststoff ist wasserlöslich.

### Bewertung:

Das verkapselte Reinigungsmittel zeigte auch hier eine deutliche Reduktion der Säurezahl bis hin zur vollständigen Entfernung nach 24 Stunden. Der Feststoff verklumpte im Versuch nicht und eine Entsorgung des Feststoffs war durch die Wasserlöslichkeit einfach möglich.

Das Lösemitteldestillat zeigt sich unverändert zum Ausgangsmaterial, mit der Ausnahme, dass die Säure entfernt wurde. Das Lösemittel kann somit weiter zur Reinigung eingesetzt werden und gewinnt durch die Säureentfernung deutlich an Wertigkeit, da korrosive Bestandteile entfernt werden.

### Versuch 6: Säurereduktion mithilfe verkapseltem Reinigungsmittel - Probe 3

Säurehaltiges DOWCLENE^{®} 1601 werden über Nacht bei 100 mbar refluxiert. In den Dampfraum der Apparatur werden Metallstreifen aus Aluminium, Stahl, Kupfer und Messing eingehängt. Am nächsten Tag wird an der Probenahmstelle im Destillat eine Probe zur Nullwertbestimmung genommen.

Das verkapselte Reinigungsmittel wird durch das Einschweißen von wasserfreiem Natriumcarbonat und Beimengen eines Antioxidants in Mowital Thin Film 100-Folie hergestellt.

Zur Reduktion der Säure wird das verkapselte Reinigungsmittel zum Sumpf hinzugefügt. Die Folie löst sich in heißem Lösemittel innerhalb von 3 bis 5 Minuten auf und gibt den Feststoff frei, der sich feindispers verteilt.

Das Lösemittel wird für weitere 6 Stunden refluxiert, wobei das Reinigungsmittel die Säure im Sumpf komplexiert. Zur Erfolgsbeurteilung werden nach 2,4 und 6 Stunden weitere Proben genommen. Die Destillationsleistung der Apparatur beträgt ca. 1/3 des Gesamt-Lösemittelvolumens pro Stunde.

### Analytische Ergebnisse:

1. Säuregehalt des Destillats (Titrationsmethode):
   - Nullwert: 13391 ppm als Essigsäure
   - Nach 2 Std (2/3fache Umwälzung des Volumens): 3724 ppm
   - Nach 4 Std (4/3fache Umwälzung des Volumens): 1529 ppm
   - Nach 6 Std (2fache Umwälzung des Volumens): 537 ppm
2. GC/FID-Analyse: Das Destillat zeigte sich an allen Probenahmezeitpunkten in gleichbleibender Qualität mit der Ausnahme, dass das Essigsäure-Signal nach 6 Stunden einen mit dem Titrationsergebnis einhergehenden Abfall in der Intensität aufwies.
3. lonenchromatographische Analyse des Destillats nach 2 Stunden: Eine zur Säurekonzentration (3724 ppm) in der Größenordnung passende Konzentration an Acetat und Formiat wurde gemessen (3300 mg/L Acetat, 1700 mg/L Formiat). Weitere Anionen wurden nicht identifiziert.
4. Optische Prüfung der Metallstreifen: Die Metallstreifen zeigten keine Beschichtung, oder ähnliche kunststoffartigen Niederschläge.
5. Beurteilung des Sumpfs: Der Sumpf zeigte feindispers verteilten, weißen Feststoff. Eine Verklumpung oder Anbackung des Feststoffs oder Kunststoffs wurde nicht beobachtet. Der Feststoff ist wasserlöslich.

### Bewertung:

Das verkapselte Reinigungsmittel zeigte eine deutliche Reduktion der Säurezahl nach bereits kurzer Versuchsdauer. Der Feststoff war nicht verklumpt und eine Entsorgung des Feststoffs war durch die Wasserlöslichkeit einfach möglich.

Das Lösemitteldestillat zeigt sich unverändert zum Ausgangsmaterial mit der Ausnahme, dass die Säure entfernt wurde. Das Lösemittel kann somit weiter zur Reinigung eingesetzt werden und gewinnt durch die Säureentfernung deutlich an Wertigkeit, da korrosive Bestandteile entfernt werden.

### Vergleichsversuch 7: Säurereduktion mithilfe unverkapselten Stabilisator

Die gleiche Menge (wie in den obigen Versuchen) an säurehaltigem DOWCLENE^{®} 1601 werden über Nacht bei 100 mbar refluxiert, wobei das Lösemittel das gleiche ist wie in den vorherigen

Versuchen. In den Dampfraum der Apparatur werden Metallstreifen aus Aluminium, Stahl, Kupfer und Messing eingehängt. Am nächsten Tag wird an der Probenahmstelle im Destillat eine Probe zur Nullwertbestimmung genommen.

Das Reinigungsmittel wird durch das Vermengen von wasserfreiem Natriumcarbonat und einem Antioxidants hergestellt. Die Mengen an wasserfreiem Natrimcarbonat und Antioxidents sind identisch im Vergleich zu den oben beschrieben Versuchen. Das Reinigungsmittel wird in Pulverform zum Sumpf zugegeben.

Das Lösemittel wird für weitere 6 Stunden refluxiert, wobei das Reinigungsmittel die Säure im Sumpf komplexiert. Zur Erfolgsbeurteilung werden nach 2,4 und 6 Stunden weitere Proben genommen. Die Destillationsleistung der Apparatur beträgt ca. 1/3 des Gesamt-Lösemittelvolumens pro Stunde.

### Analytische Ergebnisse

1. Säuregehalt des Destillats (Titrationsmethode):
   - Nullwert: 12327 ppm als Essigsäure
   - Nach 2 Std (2/3fache Umwälzung des Volumens): 10239 ppm
   - Nach 4 Std (4/3fache Umwälzung des Volumens): 9916 ppm
   - Nach 6 Std (2fache Umwälzung des Volumens): 9813 ppm
2. GC/FID-Analyse: Das Destillat zeigte sich an allen Probenahmezeitpunkten in gleichbleibender Qualität, mit der Ausnahme, dass das Essigsäure-Signal nach 6 Stunden einen mit dem Titrationsergebnis einhergehenden, geringen Abfall in der Intensität aufwies.
3. lonenchromatographische Analyse des Destillats nach 2 Stunden: Eine zur Säurekonzentration (10239 ppm) ungefähr passende Konzentration an Acetat und Formiat wurde gemessen (6900 mg/L Acetat, 3400 mg/L Formiat). Weitere Anionen wurden nicht identifiziert.
4. Optische Prüfung der Metallstreifen: Die Metallstreifen zeigten keine Beschichtung, oder ähnliche kunststoffartigen Niederschläge.
5. Beurteilung des Sumpfs: Der Sumpf zeigte feindispers verteilten, weißen Feststoff. Eine Verklumpung oder Anbackung des Feststoffs oder Kunststoffs wurde nicht beobachtet. Der Feststoff ist wasserlöslich.

### Gesamtbewertung:

Das verkapselte Reinigungsmittel zeigte eine deutlich höhere Reduktion der Säurezahl als die unverkapselte Version. Es ist daher davon auszugehen, dass die Verkapselung einen technischen Vorteil im Sinne einer kontrollierten Freisetzung des Reinigungsmittels darstellt.

## Patentansprüche

1. Verfahren zur Aufreinigung von einem alkoholhaltigen Lösemittel, **dadurch gekennzeichnet, dass** man in das alkoholhaltige Lösemittel ein Reinigungsmittel einträgt, welches in einer den Stoff im Wesentlichen vollständig umgebenden Verkapselung vorliegt, wobei die Verkapselung aus einem Material besteht, das sich in dem alkoholhaltigen Lösungsmittel auflöst und es dabei nicht zu einer Bildung von störenden Verunreinigungen kommt und wobei das alkoholhaltige Lösemittel einen modifizierten Alkohol oder Mischungen von modifizierten Alkoholen umfasst.

2. Verfahren nach Anspruch 1, **dadurch gekennzeichnet, dass** das Kapselmaterial Cellulosederivate und/oder Polyvinylbutyral umfasst.

3. Verfahren nach Anspruch 1 oder 2, **dadurch gekennzeichnet, dass** das Reinigungsmittel in der Verkapselung eine Base umfasst oder aus einer Base besteht.

4. Verfahren nach einem der Ansprüche 1 bis 3, **dadurch gekennzeichnet, dass** das Reinigungsmittel ausgewählt wird aus der Gruppe, bestehend aus Natriumcarbonat, Kaliumcarbonat, Natriumhydroxid, Kaliumhydroxid, Natriumhydrogencarbonat, Calciumhydroxid, Magnesiumcarbonat und Mischungen der vorstehenden Reinigungsmittel.

5. Verfahren nach einem der Ansprüche 1 bis 4, **dadurch gekennzeichnet, dass** das alkoholische Lösemittel nach dem Inkontaktbringen mit dem im Wesentlichen verkapselten Reinigungsmittel refluxiert wird.

6. Verfahren nach einem der Ansprüche 1 bis 5, **dadurch gekennzeichnet, dass** während dem Verfahren der Aufreinigung des alkoholhaltigen Lösemittels dem Lösemittel mindestens ein Antioxidant zugesetzt wird.

7. Verfahren nach einem der Ansprüche 1 bis 6, **dadurch gekennzeichnet, dass** das alkoholhaltige Lösemittel modifizierte Alkohole, insbesondere Glykolether-Mischungen, enthält.

8. Zusammensetzung, enthaltend ein Reinigungsmittel in einer im Wesentlichen Verkapselung aus einem Polymermaterial, enthaltend Polyvinylbutyral, **dadurch gekennzeichnet, dass** das Reinigungsmittel in der Verkapselung Natriumcarbonat, Kaliumcarbonat, Natriumhydroxid, Kaliumhydroxid und Mischungen der vorstehenden Reinigungsmittel umfasst oder daraus besteht.

9. Zusammensetzung nach Anspruch 8, **dadurch gekennzeichnet, dass** das Reinigungsmittel in der Verkapselung hygroskopisch ist.

10. Zusammensetzung nach Anspruch 8 oder 9, **dadurch gekennzeichnet, dass** das Reinigungsmittel ausgewählt wird aus der Gruppe, bestehend aus Natriumcarbonat, Kaliumcarbonat, Natriumhydroxid, Kaliumhydroxid, Natriumhydrogencarbonat, Calciumhydroxid, Magnesiumcarbonat und Mischungen der vorstehenden Reinigungsmittel.

11. Verwendung einer Zusammensetzung gemäß einem der Ansprüche 8 bis 10 zur Bearbeitung von alkoholhaltigen Lösemitteln, wobei das alkoholhaltige Lösemittel einen modifizierten Alkohol oder Mischungen von modifizierten Alkoholen umfasst.

12. Verwendung nach Anspruch 11 zur Aufarbeitung von modifizierte Alkoholen, insbesondere Glykolether-Mischungen.

13. Verfahren zur Herstellung eines Zusammensetzung gemäß einem der Ansprüche 8 bis 10, **gekennzeichnet durch** die nachfolgenden Verfahrensschritten:
i. Bereitstellung einer Folie, umfassend Polyvinylbutyral;
ii. Bereitstellung eines Reinigungsmittels;
iii. Einschweißen des Stoffs in die Folie, umfassend Polyvinylbutyral, so dass der Stoff in einer im Wesentlichen vollständig umgebenden Verkapselung vorliegt.

14. Verfahren nach Anspruch 13, **dadurch gekennzeichnet, dass** die Verkapselung durch eine Verschweißung der Folie bei einer Temperatur von mindestens 120 °C durchgeführt wird.

## Claims

1. Method for purifying an alcohol-containing solvent, **characterised in that** a cleaning agent, which is present in an encapsulation that essentially completely surrounds the substance, is introduced into the alcohol-containing solvent, wherein the encapsulation consists of a material that dissolves in the alcohol-containing solvent without forming disruptive impurities and wherein the alcohol-containing solvent comprises a modified alcohol or mixtures of modified alcohols.

2. Method according to claim 1, **characterised in that** the encapsulation material comprises cellulose derivatives and/or polyvinyl butyral.

3. Method according to claim 1 or 2, **characterised in that** the cleaning agent in the encapsulation comprises a base or consists of a base.

4. Method according to one of claims 1 to 3, **characterised in that** the cleaning agent is selected from the group consisting of sodium carbonate, potassium carbonate, sodium hydroxide, potassium hydroxide, sodium hydrogen carbonate, calcium hydroxide, magnesium carbonate and mixtures of the above cleaning agents.

5. Method according to one of claims 1 to 4, **characterised in that** the alcoholic solvent is refluxed after being brought into contact with the substantially encapsulated cleaning agent.

6. Method according to one of claims 1 to 5, **characterised in that** during the process of purifying the alcohol-containing solvent, at least one antioxidant is added to the solvent.

7. Method according to one of claims 1 to 6, **characterised in that** the alcohol-containing solvent contains modified alcohols, in particular glycol ether mixtures.

8. Composition containing a cleaning agent in a substantially encapsulation made of a polymer material containing polyvinyl butyral, **characterised in that** the cleaning agent in the encapsulation comprises or consists of sodium carbonate, potassium carbonate, sodium hydroxide, potassium hydroxide and mixtures of the above cleaning agents.

9. Composition according to claim 8, **characterised in that** the cleaning agent in the encapsulation is hygroscopic.

10. Composition according to claim 8 or 9, **characterised in that** the cleaning agent is selected from the group consisting of sodium carbonate, potassium carbonate, sodium hydroxide, potassium hydroxide, sodium hydrogen carbonate, calcium hydroxide, magnesium carbonate and mixtures of the above cleaning agents.

11. Use of a composition according to any one of claims 8 to 10 for treating alcohol-containing solvents, wherein the alcohol-containing solvent comprises a modified alcohol or mixtures of modified alcohols.

12. Use according to claim 11 for the processing of modified alcohols, in particular glycol ether mixtures.

13. Method for producing a composition according to one of claims 8 to 10, **characterised by** the following process steps:
i. providing a film comprising polyvinyl butyral;
ii. providing a cleaning agent;
iii. sealing the agent into the film comprising polyvinyl butyral so that the agent is present in a substantially completely surrounding encapsulation.

14. Method according to claim 13, **characterised in that** the encapsulation is carried out by sealing the film at a temperature of at least 120 °C.

## Revendications

1. Procédé de purification d'un solvant alcoolique , **caractérisé en ce qu'**on introduit dans le solvant alcoolique un agent nettoyant qui se présente sous la forme d'une encapsulation entourant pratiquement complètement la substance, l'encapsulation étant constituée d'un matériau qui se dissout dans le solvant alcoolique sans entraîner la formation d'impuretés gênantes et le solvant alcoolique comprenant un alcool modifié ou des mélanges d'alcools modifiés.

2. Procédé selon la revendication 1, **caractérisé en ce que** le matériau d'encapsulation comprend des dérivés de cellulose et/ou du polyvinylbutyral.

3. Procédé selon la revendication 1 ou 2, **caractérisé en ce que** le produit nettoyant dans l'encapsulation comprend une base ou est constitué d'une base.

4. Procédé selon l'une des revendications 1 à 3, **caractérisé en ce que** l'agent nettoyant est choisi dans le groupe constitué par le carbonate de sodium, le carbonate de potassium, l'hydroxyde de sodium, l'hydroxyde de potassium, l'hydrogénocarbonate de sodium, l'hydroxyde de calcium, le carbonate de magnésium et des mélanges des agents nettoyants précités.

5. Procédé selon l'une des revendications 1 à 4, **caractérisé en ce que** le solvant alcoolique est refluxé après avoir été mis en contact avec le produit nettoyant essentiellement encapsulé.

6. Procédé selon l'une des revendications 1 à 5, **caractérisé en ce qu'**au moins un antioxydant est ajouté au solvant pendant le processus de purification du solvant alcoolique.

7. Procédé selon l'une des revendications 1 à 6, **caractérisé en ce que** le solvant alcoolique contient des alcools modifiés, en particulier des mélanges d'éthers de glycol.

8. Composition contenant un agent nettoyant dans une encapsulation essentiellement constituée d'un matériau polymère contenant du polyvinylbutyral, **caractérisée en ce que** l'agent nettoyant dans l'encapsulation comprend ou est constitué de carbonate de sodium, carbonate de potassium, hydroxyde de sodium, hydroxyde de potassium et mélanges des agents nettoyants précités .

9. Composition selon la revendication 8, **caractérisée en ce que** l'agent nettoyant dans l'encapsulation est hygroscopique.

10. Composition d' e selon la revendication 8 ou 9, **caractérisée en ce que** l'agent nettoyant est choisi dans le groupe constitué par le carbonate de sodium, le carbonate de potassium, l'hydroxyde de sodium, l'hydroxyde de potassium, l'hydrogénocarbonate de sodium, l'hydroxyde de calcium, le carbonate de magnésium et les mélanges des agents nettoyants précités.

11. Utilisation d'une composition selon l'une des revendications 8 à 10 pour le traitement de solvants contenant de l'alcool, le solvant contenant de l'alcool comprenant un alcool modifié ou des mélanges d'alcools modifiés.

12. Utilisation selon la revendication 11 pour le traitement d'alcools modifiés, en particulier de mélanges d'éthers de glycol.

13. Procédé de fabrication d'une composition selon l'une des revendications 8 à 10, **caractérisé par** les étapes suivantes :
i. fourniture d'un film comprenant du polyvinylbutyral;
ii. fourniture d'un agent nettoyant;
iii. soudage l'agent dans le film comprenant du polyvinylbutyral, de sorte que l'agent se trouve dans une encapsulation qui l'entoure pratiquement complètement.

14. Procédé selon la revendication 13, **caractérisé en ce que** l'encapsulation est réalisée par soudage du film à une température d'au moins 120 °C.
